# EUROPEAN PATENT APPLICATION

(11) **EP 1 749 473 A1**
(43) Date of publication of application: **07.02.2007**
(21) Application number: 05741511.9
(22) Date of filing: 23.05.2005
(51) Int. Cl.: A61B 1/00

(54) **POSITION INFORMATION DETECTING DEVICE AND POSITION INFORMATION DETECTING SYSTEM**

(30) Priority: 26.05.2004 JP 2004156408
(71) Applicant: Olympus Corporation, Tokyo 151-0072 (JP)
(72) Inventor: MINAI, Tetsuo, 1920919 (JP); MATSUI, Akira, 1910001 (JP)
(74) Representative: von Hellfeld, Axel
(86) International application number: PCT/JP2005/009381
(87) International publication number: WO 2005/115219

(57) **Abstract**

To realize a technique for calculating a positional relationship between a target coordinate axis fixed with respect to a detection target and a reference coordinate axis defined independently of motion and the like of the detection target, a first and a second linear magnetic fields in known directions on the reference coordinate axis are generated and a direction of the first and the second linear magnetic fields on the target coordinate axis are detected by a magnetic field sensor installed in an capsule endoscope (2), which is the detection target. By comparing the directions, which are detected by the magnetic field sensor, of the first and the second linear magnetic fields on the target coordinate axis and the known directions of the first and the second linear magnetic fields on the reference coordinate axis, a deviation of the direction of the target coordinate axis with respect to the reference coordinate axis is detected. Further, a positional relationship of an origin of the target coordinate axis with respect to an origin of the reference coordinate axis is calculated by generating diffuse magnetic field, other than the first and the second magnetic fields, whose strength decreases corresponding to a distance.

## Description

### TECHNICAL FIELD

The present invention relates to a technique for detecting a positional relationship between a target coordinate axis fixed with respect to a detection target and a reference coordinate axis defined independently of motion of the detection target.

### BACKGROUND ART

In a field of endoscopes, a swallowable type capsule endoscope has been proposed in recent years. The capsule endoscope has an imaging function and a radio transmission function. During a period from when the capsule endoscope is swallowed from a mouth of a subject for an observation (inspection) until when the capsule endoscope is naturally discharged, the capsule endoscope travels through inside a body cavity, i.e., inside organs such as a gaster and a small intestine, and sequentially images inside the organs, while following peristaltic motion of the organs.

An image obtained inside the body by the capsule endoscope is sequentially transmitted to outside through radio transmission and stored in a memory provided outside, while the capsule endoscope travels through inside the body cavity. The subject can freely move during the period from when the capsule endoscope is swallowed until when the capsule endoscope is discharged, since the subject carries around a receiver that has the radio transmission function and a memory function. After the capsule endoscope is discharged, a diagnosis can be made by a doctor or a nurse by displaying an image, which is based on the image data stored in the memory, of the organs on a display (For example, see Patent Document 1).

In a conventional capsule endoscope system, there is proposed a configuration in which power is supplied to an capsule endoscope from an external device through radio signals to allow the capsule endoscope to be driven for a long time after the capsule endoscope is inserted into the subject. The capsule endoscope is required to have a light and miniaturized configuration since the capsule endoscope is to be inserted into the subject. Hence, size and weight of a battery to be installed in the capsule endoscope needs to be reduced, and accordingly, it is difficult to install a battery having capacity required to drive the capsule endoscope for a long time.

Therefore, the capsule endoscope further has a battery and the like having a charging function and a receiver such as a receiving antenna for receiving radio signals transmitted from outside. The capsule endoscope regenerates the power by receiving the radio signals transmitted from outside, and the capsule endoscope uses the power regenerated as driving power thereof. Consequently, it becomes unnecessary to install a large capacity battery in the capsule endoscope, and an capsule endoscope operated for a long time inside the subject can be realized.

Patent Document 1: Japanese Patent Application Laid-open No. 2003-19111

### DISCLOSURE OF INVENTION

### PROBLEM TO BE SOLVED BY THE INVENTION

However, the conventional capsule endoscope system has a problem in which the radio signals transmitted from outside to the capsule endoscope cannot be transmitted effectively. Hereinafter, the problem is explained in detail.

When the capsule endoscope acquires the driving power from the radio signals transmitted from outside, a receiving antenna provided in the capsule endoscope is arranged as being fixed with respect to the capsule endoscope, so as to be arranged at a predetermined position on coordinate axes (hereinafter referred to as "target coordinate axes") fixed with respect to the capsule endoscope. The capsule endoscope travels inside the subject, while rotating on an axis of a traveling direction of the capsule endoscope due to friction and the like between the capsule endoscope and an inner wall of organs forming a passage, and while changing the traveling direction thereof corresponding to the passage.

Hence, a positional relationship between exterior coordinate axes, e.g., coordinate axes fixed with respect to an exterior surface of the subject (hereinafter referred to as "reference coordinate axes"), and the target coordinate axes fixed with respect to the capsule endoscope changes randomly along with the traveling of the capsule endoscope. A direction in which the receiving antenna fixed on the target coordinate axes can most effectively receive the radio signals changes randomly on the reference coordinate axes. Therefore, a problem, in which the receiving antenna cannot receive most of the radio signals transmitted from a transmitting antenna, is caused depending on a certain positional relationship between the capsule endoscope and the transmitting antenna fixed on the reference coordinate axes.

In order to alleviate the aforementioned problem, it is important to determine an orientation direction and a position of the capsule endoscope inside the subject, thus it is important to determine a positional relationship between the reference coordinate axes defined outside of the subject and the target coordinate axes, which changes randomly inside the subject, of the capsule endoscope. However, an effective technique for determining the aforementioned positional relationship is not proposed at least in publicly known techniques so far.

The present invention is achieved in view of the foregoing, and an object of the present invention is to provide a technique that calculates the positional relationship between the target coordinate axes fixed with respect to the detection target such as the capsule endoscope and the reference coordinate axes defined independently of the traveling of the detection target and the like.

### MEANS FOR SOLVING PROBLEM

In order to solve the aforementioned problem and to achieve the object, a positional relationship detecting apparatus as set forth in claim 1 detects a positional relationship between a target coordinate axis fixed with respect to a detection target and a reference coordinate axis defined independently of motion of the detection target. The positional relationship detecting apparatus includes an orientation calculator that calculates an orientation of the target coordinate axis with respect to the reference coordinate axis based on correspondences. One correspondence is between a direction of a first linear magnetic field having a predetermined direction on the target coordinate axis and a direction of the first linear magnetic field on the reference coordinate axis, and other correspondence is between a direction of a second linear magnetic field different from the first linear magnetic field on the target coordinate axis and a direction of the second linear magnetic field on the reference coordinate axis.

The positional relationship detecting apparatus as set forth in claim 2 further includes a first linear magnetic field generator that is arranged at a predetermined position on the reference coordinate axis and generates the first linear magnetic field, and a second linear magnetic field generator that is arranged at a predetermined position on the reference coordinate axis and generates the second linear magnetic field. The orientation calculator calculates the orientation of the target coordinate axis with respect to the reference coordinate axis based on the directions, which are detected by the detection target, of the first and the second linear magnetic fields on the target coordinate axis and the directions of the first and the second linear magnetic fields on the predetermined reference coordinate axis.

The positional relationship detecting apparatus as set forth in claim 3 further includes a second linear magnetic field generator that is arranged at a predetermined position on the reference coordinate axis and generates the second linear magnetic field, and a magnetic field sensor that detects the direction of the first linear magnetic field on the reference coordinate axis. The orientation calculator calculates the orientation of the target coordinate axis with respect to the reference coordinate axis based on the direction, which is detected by the magnetic field sensor, of the first linear magnetic field on the reference coordinate axis, the direction of the second linear magnetic field on the predetermined reference coordinate axis, and directions, which are detected by the detection target, of the first and the second linear magnetic fields on the target coordinate axis.

The positional relationship detecting apparatus as set forth in claim 4, 5, or 6 further includes a position calculator that calculates an origin of the target coordinate axis with respect to the reference coordinate axis based on a direction of a diffuse magnetic field whose direction has positional dependence at a position of the detection target, and the orientation, which is calculated by the orientation calculator, of the target coordinate axis with respect to the reference coordinate axis.

In the positional relationship detecting apparatus as set forth in claim 7 or 8, the second linear magnetic field has a property in which a magnetic field strength decreases corresponding to a distance from the second linear magnetic field generator, and the position calculator calculates the origin of the target coordinate axis with respect to the reference coordinate axis by calculating and using a distance between the detection target and the second linear magnetic field generator based on the magnetic field strength of the second linear magnetic field at the position of the detection target.

A positional relationship detecting system as set forth in claim 9 includes a detection target defined with a predetermined target coordinate axis; and a positional relationship detecting apparatus that detects a positional relationship between the target coordinate axis and a reference coordinate axis defined independently of motion of the detection target. The detection target includes a magnetic field sensor that detects a magnetic field generated in a region where the detection target is positioned, and a radio signal transmitter that transmits a radio signal containing information on the magnetic field detected by the magnetic field sensor. The positional relationship detecting apparatus includes a magnetic field generator that generates a magnetic field in the region where the detection target is positioned, and an orientation calculator that calculates an orientation of the target coordinate axis with respect to the reference coordinate axis based on the radio signal transmitted from the detection target.

In the positional relationship detecting system as set forth in claim 10, the magnetic field generator includes a first linear magnetic field generator that is arranged at a predetermined position on the reference coordinate axis and generates a first magnetic field in a predetermined direction, and a second linear magnetic field generator that is arranged at a predetermined position on the reference coordinate axis and generates a second linear magnetic field different from the first linear magnetic field. The orientation calculator calculates the orientation of the target coordinate axis with respect to the reference coordinate axis based on correspondences, one correspondence between a direction of the first linear magnetic field on the target coordinate axis and a direction of the first linear magnetic field on the reference coordinate axis, other correspondence between a direction of the second linear magnetic field on the target coordinate axis and a direction of the second linear magnetic field on the reference coordinate axis.

In the positional relationship detecting system as set forth in claim 11 or 12, the magnetic field generator further includes a diffuse-magnetic-field generator that generates a diffuse magnetic field whose direction has positional dependence, and the positional relationship detecting apparatus includes a position calculator that calculates an origin of the target coordinate axis on the reference coordinate axis by using the positional dependence of the direction of the diffuse magnetic field.

### EFFECT OF THE INVENTION

A positional relationship detecting apparatus and a positional relationship detecting system according to the present invention has an orientation calculator that calculates an orientation of target coordinate axes with respect to reference coordinate axes based on a correspondence among orientations of a plurality of linear magnetic fields. Consequently, the positional relationship detecting apparatus and the positional relationship detecting system can detect the orientation of the target coordinate axes with respect to the reference coordinate axes even if an orientation direction and the like of the detection target change during traveling of the detection target.

The positional relationship detecting apparatus and the positional relationship detecting system according to the present invention has a position calculator that calculates an origin of the target coordinate axes with respect to the reference coordinate axes based on a detection result of diffuse magnetic field having positional dependence with the traveling direction of the detection target. Consequently, the positional relationship detecting apparatus and the positional relationship detecting system can detect the origin of the target coordinate axes with respect to the reference coordinate axes even if the origin of the target coordinate axes moves during the traveling of the detection target.

### BRIEF DESCRIPTION OF DRAWINGS

FIG. 1 is a schematic drawing of an overall configuration of a positional relationship detecting system according to a first embodiment;
FIG. 2 is a block diagram of a configuration of an capsule endoscope provided in the positional relationship detecting system;
FIG. 3 is a schematic drawing of a configuration of a second linear magnetic field generator and a diffuse-magnetic-field generator provided in the positional relationship detecting system;
FIG. 4 is a block diagram of a configuration of a processor provided in the positional relationship detecting system;
FIG. 5 is a schematic drawing of a direction of first linear magnetic field generated by a first linear magnetic field generator provided in the positional relationship detecting system;
FIG. 6 is a schematic drawing of a direction of second linear magnetic field generated by the second linear magnetic field generator provided in the positional relationship detecting system;
FIG. 7 is a schematic drawing of a direction of diffuse magnetic field generated by the diffuse-magnetic-field generator;
FIG. 8 is a schematic drawing for explaining a direction detecting mechanism according to which an orientation of target coordinate axes is detected by the positional relationship detecting system with respect to reference coordinate axes;
FIG. 9 is a schematic drawing for explaining an origin detecting mechanism according to which an origin of the target coordinate axes is detected by the positional relationship detecting system with respect to the reference coordinate axes;
FIG. 10 is a schematic drawing for explaining the origin detecting mechanism according to which the origin of the target coordinate axes is detected by the positional relationship detecting system with respect to the reference coordinate axes;
FIG. 11 is a schematic drawing of an overall configuration of a positional relationship detecting system according to a second embodiment; and
FIG. 12 is a block diagram of a configuration of a processor provided in the positional relationship detecting system according to the second embodiment.

### EXPLANATIONS OF LETTERS OR NUMERALS

- 1: Subject
- 2: Capsule endoscope
- 3: Positional relationship detecting apparatus
- 4: Display device
- 5: Portable recording medium
- 7a-7d: Receiving antenna
- 8a-8d: Transmitting antenna
- 9: First linear magnetic field generator
- 10: Second linear magnetic field generator
- 11: Diffuse-magnetie-field generator
- 12: Processor
- 14: In-vivo information acquiring unit
- 15: Signal processing unit
- 16: Magnetic field sensor
- 17: Amplifier
- 18: A/D converter
- 19: Radio transmitting unit
- 20: Switching unit
- 21: Timing generator
- 22: LED
- 23: LED driving circuit
- 24: CCD
- 25: CCD driving circuit
- 26: Transmitting circuit
- 27: Transmitting antenna
- 28: Receiving antenna
- 29: Power regenerating circuit
- 30: Voltage step-up circuit
- 31: Capacitor
- 32: Coil
- 33: Current source
- 34: Coil
- 35: Current source
- 37: Receiving antenna selector
- 38: Receiving circuit
- 39: Signal processing unit
- 40: Orientation calculator
- 41: Position calculator
- 42: Magnetic-field line orientation database
- 43: Memory
- 44: Oscillator
- 46: Amplifying circuit
- 47: Transmitting antenna selector
- 48: Selection controller
- 49: Power supply unit
- 51: Curved surface
- 53: Positional relationship detecting apparatus
- 54: Earth magnetism sensor
- 55: Processor

- 56: Earth magnetism orientation calculator

### BEST MODE(S) FOR CARRYING OUT THE INVENTION

Hereinafter, exemplary embodiments of a positional relationship detecting apparatus and a positional relationship detecting system according to the present invention are explained. It should be noted that the accompanying drawings are merely schematic, and relation between width and thickness of each portion, thickness ratio of one portion to another, and the like may be different in an actual apparatus and a system. The dimensional relations and the ratio may be different from one drawing to another.

### First Embodiment

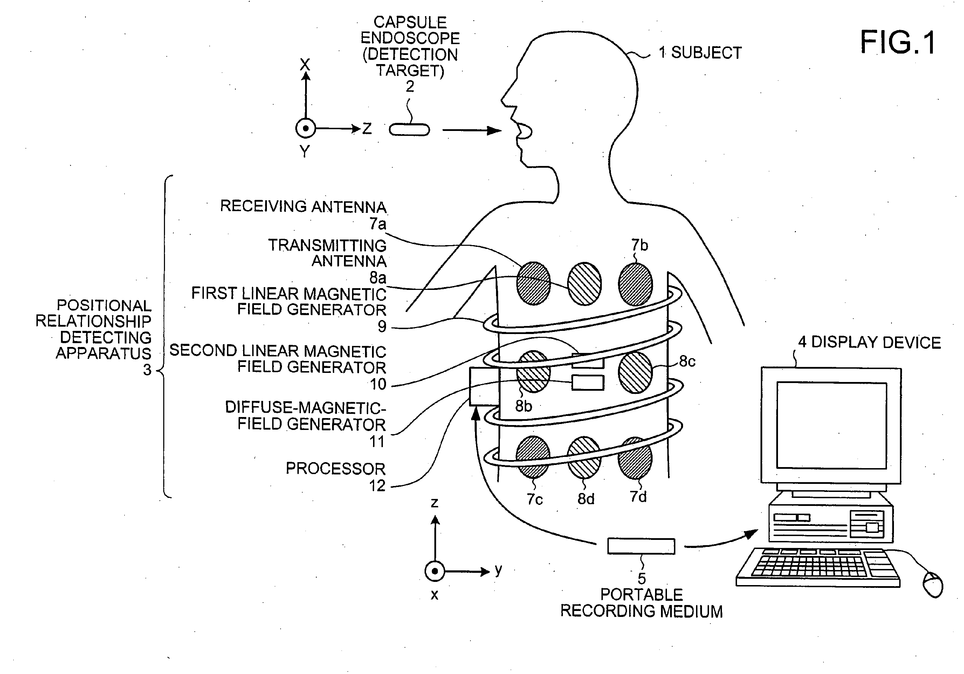

A positional relationship detecting system according to a first embodiment is explained. FIG. 1 is a schematic drawing of an overall configuration of the positional relationship detecting system according to the first embodiment. As shown in FIG. 1, the positional relationship detecting system according to the first embodiment has an capsule endoscope 2, a positional relationship detecting apparatus 3, a display device 4, and a portable recording medium 5. The capsule endoscope 2 is inserted into a subject 1 and travels along a passage. The positional relationship detecting apparatus 3 performs radio transmission between the positional relationship detecting apparatus 3 and the capsule endoscope 2, and detects a positional relationship between target coordinate axes fixed with respect to the capsule endoscope 2 and reference coordinate axes fixed with respect to the subject 1. The display device 4 displays contents of radio signals transmitted from the capsule endoscope 2 and received by the positional relationship detecting apparatus 3. The portable recording medium 5 serves for information transfer between the positional relationship detecting apparatus 3 and the display device 4. As shown in FIG. 1, the target coordinate axes and the reference coordinate axes are set in the first embodiment. The target coordinate axes are defined by the X-, Y-, and Z-axes and fixed with respect to the capsule endoscope 2. The reference coordinate axes are defined by the x-, y-, and z-axes and defined independently of motion of the capsule endoscope 2, and specifically, the reference coordinate axes are fixed with respect to the subject 1. The positional relationship detecting system detects a positional relationship of the target coordinate axes with respect to the reference coordinate axes by utilizing a mechanism explained hereinafter.

The display device 4 displays, for example, a subject interior image obtained through imaging by the capsule endoscope 2 and received by the positional relationship detecting apparatus 3, and has a configuration such as a work station displaying an image based on data acquired from the portable recording medium 5. Specifically, the display device 4 may directly display the image and the like by a cathode ray tube (CRT) display, a liquid crystal display, and the like, or may output the image to other medium as in a printer.

The portable recording medium 5 is detachable with respect to a processor 12 described hereinafter and the display device 4, and has a configuration that can output and record information when the portable recording medium 5 is attached to one of the processor 12 and the display device 4. Specifically, the portable recording medium 5 is attached to the processor 12 and stores the subject interior image and the positional relationship of the target coordinate axes with respect to the reference coordinate axes, while the capsule endoscope 2 travels inside a body cavity of the subject 1. After the capsule endoscope 2 is discharged from the subject 1, the portable recording medium 5 is removed from the processor 12 and attached to the display device 4, and the display device 4 reads out data recorded in the portable recording medium 5. Unlike when the processor 12 and the display device 4 are connected to each other through a cable, the subject 1 can freely move while the capsule endoscope 2 travels inside the subject 1, since the data are transferred between the processor 12 and the display device 4 by the portable recording medium 5 such as a Compact Flash® memory and the like.

The capsule endoscope 2 is explained. The capsule endoscope 2 functions as an example of a detection target in claims. Specifically, the capsule endoscope 2 is inserted into the subject 1, and acquires in-vivo information and transmits radio signals containing the acquired in-vivo information to outside, while traveling through inside the subject 1. Further, the capsule endoscope 2 has a magnetic field detecting function for detecting a positional relationship described hereinafter, and has a configuration in which driving power thereof is supplied from outside. Specifically, the capsule endoscope 2 receives the radio signals transmitted from outside and regenerates the received radio signals as the driving power thereof.

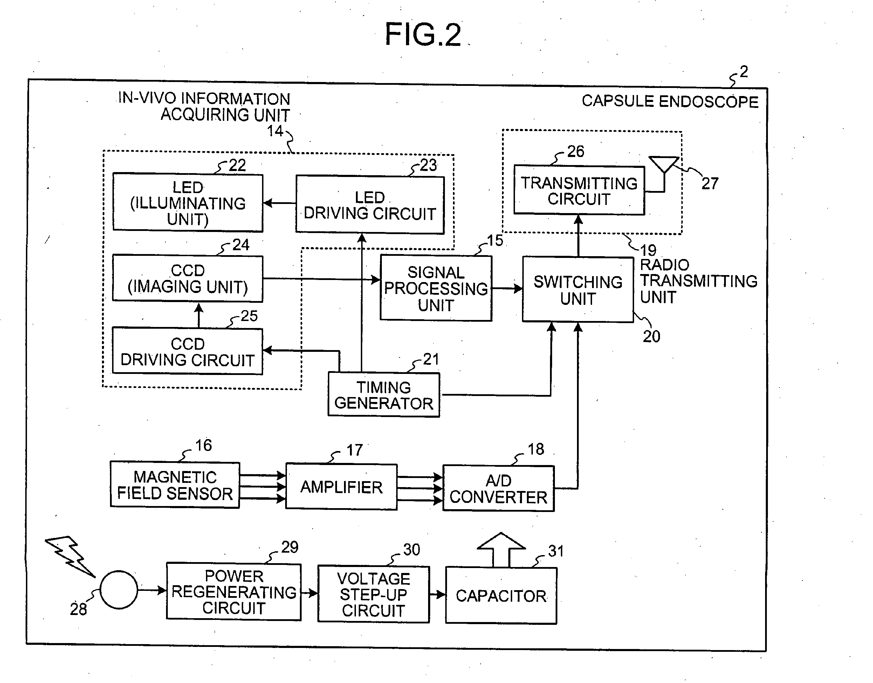

FIG. 2 is a block diagram of a configuration of the capsule endoscope 2. As shown in FIG. 2, the capsule endoscope 2 has an in-vivo information acquiring unit 14 that acquires the in-vivo information and a signal processing unit 15 that performs a predetermined processing on the acquired in-vivo information, as a mechanism for acquiring the in-vivo information. Further, the capsule endoscope 2 has a magnetic field sensor 16, an amplifier 17, and an analog/digital (A/D) converter 18, as a magnetic field detecting mechanism. The magnetic field sensor 16 detects magnetic field, and outputs electronic signals corresponding to the detected magnetic field. The amplifier 17 amplifies the output electronic signals. The A/D converter 18 converts the electronic signals output from the amplifier 17 to digital signals.

The in-vivo information acquiring unit 14 serves to acquire the in-vivo information, and in the first embodiment, the in-vivo information acquiring unit 14 serves to acquire a subject interior image, which is the image data of the subject interior. Specifically, the in-vivo information acquiring unit 14 has a light-emitting diode (LED) 22 that functions as an illuminating unit, an LED driving circuit 23 that controls driving of the LED 22, a charge-coupled device (CCD) 24 that functions as an imaging unit picking up at least one portion of a region illuminated by the LED 22, and a CCD driving circuit 25 that controls driven state of the CCD 24. In practice, the illuminating unit and the imaging unit do not need to include the LED and the CCD, respectively, and a complementary metal oxide semiconductor (CMOS) and the like can be used as the imaging unit.

The magnetic field sensor 16 detects an orientation and a strength of the magnetic field generated in a region where the capsule endoscope 2 is located. Specifically, the magnetic field sensor 16 includes a Magneto Impedance (MI) sensor, for example. The MI sensor employs a FeCoSiB amorphous wire as a magneto-sensitive medium. When a high-frequency electric current is applied to the magneto-sensitive medium, a magnetic impedance of the magneto-sensitive medium dramatically changes due to an external magnetic field. This phenomenon is called MI effect. The MI sensor utilizes the MI effect to detect the magnetic field strength. The magnetic field sensor 16 may be configured with a Magnetic Resistance Element (MRE), a Giant Magnetoresistive (GMR) magnetic sensor, and the like, in place of the MI sensor.

As shown in FIG. 1, the target coordinate axes defined by the X-, Y-, and Z-axes are employed as the coordinate axes of the capsule endoscope 2, which is the detection target, in the first embodiment. In accordance with the target coordinate axes, the magnetic field sensor 16 detects X, Y, and Z components of the magnetic field strength of the magnetic field generated in the region where the capsule endoscope 2 is positioned, and the magnetic field sensor 16 outputs electronic signals corresponding to the magnetic field strength in each direction. The components of the magnetic field strength, which are detected by the magnetic field sensor 16, on the target coordinate axes are transmitted to the positional relationship detecting apparatus 3 through a radio transmitting unit 19 described hereinafter. Then, the positional relationship detecting apparatus 3 calculates the positional relationship between the target coordinate axes and the reference coordinate axes based on values of the magnetic field components detected by the magnetic field sensor 16.

The capsule endoscope 2 has a transmitting circuit 26, and a transmitting antenna 27. Further, the capsule endoscope 2 includes the radio transmitting unit 19 for performing radio transmission with respect to outside, and a switching unit 20 which switches signals output to the radio transmitting unit 19 as appropriate between signals output from the signal processing unit 15 and signals output from the A/D converter 18. Further, the capsule endoscope 2 has a timing generator 21 for synchronizing driving timings of the in-vivo information acquiring unit 14, the signal processing unit 15, and the switching unit 20.

The capsule endoscope 2 has a receiving antenna 28, a power regenerating circuit 29, a voltage step-up circuit 30, and a capacitor 31, as a mechanism for receiving power feeding radio signals transmitted from outside. The power regenerating circuit 29 regenerates power from the radio signals received through the receiving antenna 28. The voltage step-up circuit 30 increases voltage of power signals output from the power regenerating circuit 29. The capacitor 31 accumulates the power signals with voltage changed to a predetermined voltage by the voltage step-up circuit 30, and supplies the power signals as the driving power of other aforementioned elements.

The receiving antenna 28 includes, for example, a loop antenna. The loop antenna is secured at a predetermined position in the capsule endoscope 2. Specifically, the loop antenna is arranged at a predetermined position and in a predetermined orientation direction on the target coordinate axes fixed with respect to the capsule endoscope 2.

The positional relationship detecting apparatus 3 is explained. As shown in FIG. 1, the positional relationship detecting apparatus 3 has receiving antennas 7a to 7d that receive the radio signals transmitted from the capsule endoscope 2, transmitting antennas 8a to 8d that transmit the power feeding radio signals to the capsule endoscope 2, a first linear magnetic field generator 9 that generates first linear magnetic field, a second linear magnetic field generator 10 that generates second linear magnetic field, a diffuse-magnetic-field generator 11 that generates diffuse magnetic field, and a processor 12 that performs a predetermined processing on the radio signals and the like received through the receiving antennas 7a to 7d.

The receiving antennas 7a to 7d receive the radio signals transmitted from the radio transmitting unit 19 provided in the capsule endoscope 2. Specifically, the receiving antennas 7a to 7d include loop antennas and the like, and have a function of transmitting the received radio signals to the processor 12.

The transmitting antennas 8a to 8d transmit the radio signals generated by the processor 12 to the capsule endoscope 2. Specifically, the transmitting antennas 8a to 8d include loop antennas and the like electrically connected to the processor 12.

It should be noted that specific configurations of the receiving antennas 7a to 7d, the transmitting antennas 8a to 8d, the first linear magnetic field generator 9 described hereinafter, and the like are not limited to the configurations shown in FIG. 1. FIG. 1 shows the aforementioned configurations only schematically. Hence, the number, position, specific shapes, and the like of the receiving antennas 7a to 7d and the like are not limited to the configurations shown in FIG. 1, and any configurations may be employed.

The first linear magnetic field generator 9 generates linear magnetic field in a predetermined direction, inside the subject 1. Here, the "linear magnetic field" is a magnetic field which has a magnetic field component in practically only one direction in at least a predetermined space region, and in the first embodiment, the predetermined space region is a space region in which the capsule endoscope 2 inside the subject 1 may be positioned. Specifically, as shown in FIG. 1, the first linear magnetic field generator 9 has a coil formed as to cover a torso of the subject 1 and a current source (not shown) that supplies a predetermined current to the coil, and the first linear magnetic field generator 9 generates the linear magnetic field in the space region inside the subject 1 by running the predetermined current through the coil. The direction of the first linear magnetic field may arbitrary be selected. In the first embodiment, the first linear magnetic field is linear magnetic field that travels in a direction of the z-axis of the reference coordinate axes, which is fixed with respect to the subject 1.

The second linear magnetic field generator 10 generates second linear magnetic field, which is linear magnetic field that travels in a direction different from the direction of the first linear magnetic field. Unlike the first linear magnetic field generator 9 and the second linear magnetic field generator 10, the diffuse-magnetic-field generator 11 serves to generate diffuse magnetic field whose direction has positional dependence, and in the first embodiment, the diffuse-magnetic-field generator 11 serves to generate magnetic field that diffuses with distance from the diffuse-magnetic-field generator 11.

In the first embodiment, the first linear magnetic field generator 9, the second linear magnetic field generator 10, and the diffuse-magnetic-field generator 11 generate the magnetic fields at different times. In other words, in the first embodiment, the first linear magnetic field generator 9, the second linear magnetic field generator 10, and the diffuse-magnetic-field generator 11 do not generate the magnetic fields simultaneously, but generate magnetic fields in a predetermined sequence, and the magnetic field sensor 16 provided in the capsule endoscope 2 detects the first linear magnetic field, the second linear magnetic field, and the diffuse magnetic field separately.

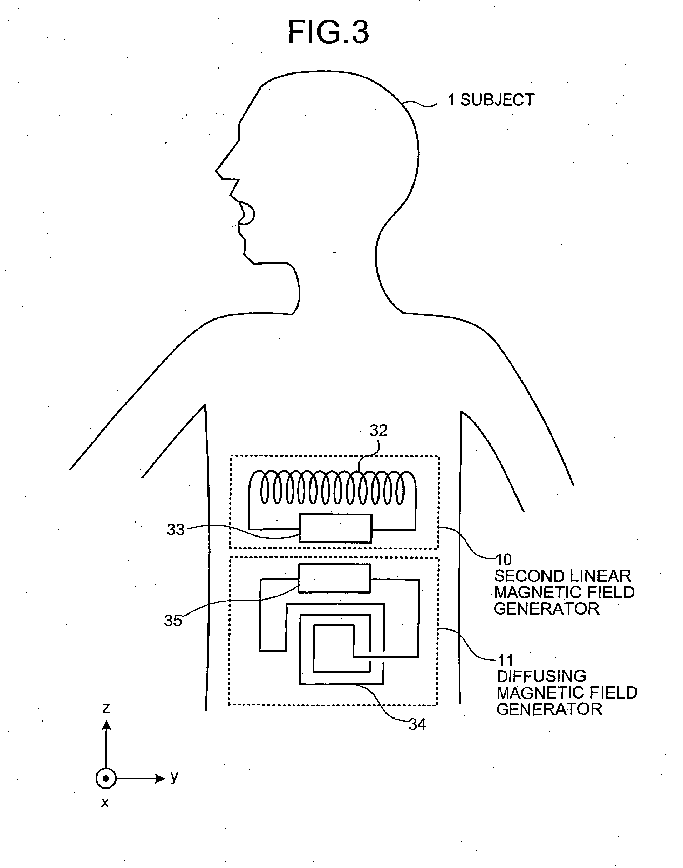

FIG. 3 is a schematic drawing of a specific configuration of the second linear magnetic field generator 10 and the diffuse-magnetic-field generator 11. As shown in FIG. 3, the second linear magnetic field generator 10 has a coil 32 and a current source 33. The coil 32 extends in the y-axis direction of the reference coordinate axes, and a cross section thereof is parallel to an xz plane. The current source 33 supplies current to the coil 32. The diffuse-magnetic-field generator 11 has a coil 34 and a current source 35 that supplies current to the coil 34. The coil 32 is arranged so as to generate the magnetic field whose direction is in a previously determined direction, and in the present first embodiment, the coil 32 is arranged so that the direction of the linear magnetic field generated by the coil 32 is in the y-axis direction of the reference coordinate axes. The coil 34 is secured at a position where the coil 34 can generate the diffuse magnetic field whose direction is the same as the magnetic field direction stored in a magnetic-field line orientation database 42 described later.

The processor 12 is explained. The processor 12 performs the radio transmission between the processor 12 and the capsule endoscope 2, and detects the orientation direction, the position, and the like, of the capsule endoscope 2 based on the received radio signals, in other words, calculates the positional relationship between the target coordinate axes fixed with respect to the capsule endoscope 2 and the reference coordinate axes fixed with respect to the subject 1.

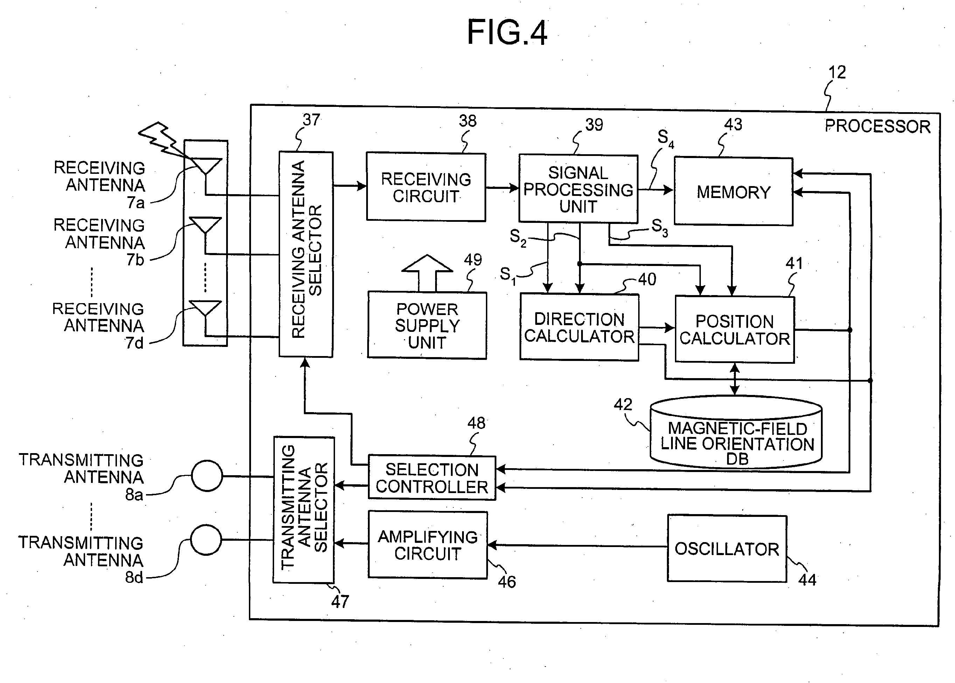

FIG. 4 is a block diagram of a specific configuration of the processor 12. Firstly, as shown in FIG. 4, the processor 12 has a mechanism for extracting the subject interior image data from the radio signals transmitted from the capsule endoscope 2. Specifically, the processor 12 has a receiving antenna selector 37, a receiving circuit 38, and a signal processing unit 39. The receiving antenna selector 37 selects an antenna, which is appropriate for receiving the radio signals, from the plurality of the receiving antennas 7a to 7d. The receiving circuit 38 performs a processing such as demodulation on the radio signals received through the receiving antenna 7 selected by the receiving antenna selector 37. The signal processing unit 39 extracts information on the detected magnetic field, the subject interior image, and the like from the radio signals on which the processing is performed.

Secondly, the processor 12 has a mechanism for calculating the positional relationship of the target coordinate axes with respect to the reference coordinate axes fixed with respect to the subject 1, by using a detection result, which is transmitted from the capsule endoscope 2, of the magnetic field generated in the region where the capsule endoscope 2 is positioned. Specifically, the processor 12 has an orientation calculator 40, a position calculator 41, and the magnetic-field line orientation database 42. The orientation calculator 40 calculates the orientation of the target coordinate axes with respect to the reference coordinate axes based on magnetic field signals S1 and S2 output from the signal processing unit 39. The position calculator 41 calculates an origin of the target coordinate axes with respect to the reference coordinate axes, by using orientation information on the orientation of the target coordinate axes output from the orientation calculator 40, the magnetic field signals S2 and S3 output from the signal processing unit 39, and the like. The magnetic-field line orientation database 42 stores information on orientations of the magnetic field lines used for the calculation in the position calculator 41.

The processor 12 further has a memory 43 that stores the extracted subject interior image and the positional relationship of the target coordinate axes with respect to the reference coordinate axes. The memory 43 writes the information in the portable recording medium 5 shown in FIG. 1.

Further, the processor 12 has a mechanism for transmitting the radio signals to the capsule endoscope 2. Specifically, the processor 12 has an oscillator 44 that determines a frequency of the radio signals to be transmitted, an amplifying circuit 46 that amplifies strength of the radio signals output from the oscillator 44, and a transmitting antenna selector 47 that is used for the transmission of the radio signals. The aforementioned configurations are employed to supply power to the capsule endoscope 2 from outside. The positional relationship detecting system according to the first embodiment allows the capsule endoscope 2 to be driven inside the subject 1 for a long time, by supplying the driving power of the capsule endoscope 2 from outside.

The processor 12 has a selection controller 48 that controls an antenna selecting operation of the receiving antenna selector 37 and the transmitting antenna selector 47 based on the results of calculation at the orientation calculator 40 and the position calculator 41. Specifically, the selection controller 48 calculates an orientation direction and a position of the transmitting antenna 27 and the receiving antenna 28, which are provided in the capsule endoscope 2, on the reference coordinate axes based on the orientation of the target coordinate axes calculated by the orientation calculator 40 and the origin of the target coordinate axes calculated by the position calculator 41. Then, the selection controller 48 commands the transmitting antenna selector 47 and the receiving antenna selector 37 to select the transmitting antenna 8 and the receiving antenna 7, which can most effectively transmit or receive the radio signals with respect to the calculated orientation direction and the position, and the selection controller 48 commands the transmitting antenna selector 47 and the receiving antenna selector 37 to switch to the selected antennas.

The processor 12 has a power supply unit 49 that supplies the driving power of each element of the processor 12. The processor 12 is configured with the aforementioned elements. By realizing each function of the elements, the processor 12 calculates the positional relationship of the target coordinate axes with respect to the reference coordinate axes based on the magnetic fields detected by the capsule endoscope, in addition to acquiring the subject interior image obtained by the capsule endoscope 2 and in addition to transmitting the radio signals to be regenerated as the driving power for the capsule endoscope 2.

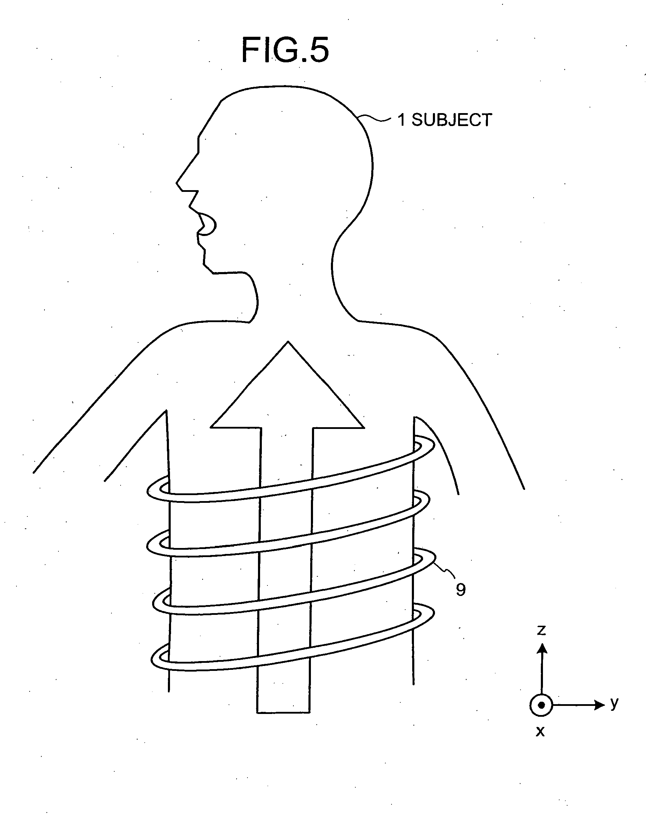

The magnetic fields generated by the first linear magnetic field generator 9, the second linear magnetic field generator 10, and the diffuse-magnetic-field generator 11 in a space in which the subject 1 resides are explained as basis of the operation to calculate the positional relationship of the target coordinate axes fixed with respect to the capsule endoscope 2. FIG. 5 is a schematic drawing of the first linear magnetic field generated by the first linear magnetic field generator 9. As shown in FIG. 5, the coil of the first linear magnetic field generator 9 is arranged so as to wind around the torso of the subject 1, and to extend in the z-axis direction of the reference coordinate axes. Therefore, the first linear magnetic field generated inside the subject 1 by the first linear magnetic field generator 9 forms magnetic field lines running in the z-axis direction of the reference coordinate axes. Thus, the first linear magnetic field generated by the first linear magnetic field generator 9 may be used as an indicator representing the direction of the z-axis of the reference coordinate axes inside the subject 1. The capsule endoscope 2 may detect the first linear magnetic field generated by the first linear magnetic field generator 9 based on the target coordinate axes. Then, the z-axis direction on the target coordinate axes can be detected as described later.

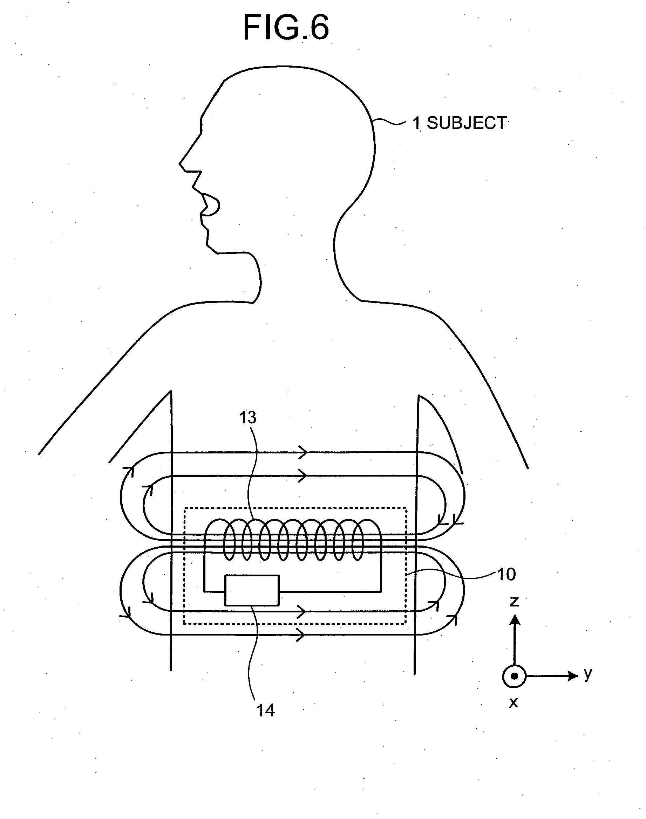

FIG. 6 is a schematic drawing of the second linear magnetic field generated by the second linear magnetic field generator 10. Since the second linear magnetic field generator 10 extends in the y-axis direction of the reference coordinate axes as explained hereinbefore, the second linear magnetic field to be generated forms magnetic field lines parallel to the y-axis direction as shown in FIG. 6. Unlike the first linear magnetic field generator 9, the second linear magnetic field generator 10 has the coil 32 arranged outside of the subject 1; therefore, the magnetic field strength of the second linear magnetic field gradually decreases with distance from the second linear magnetic field generator 10, inside the subject 1. Due to such a property of the second linear magnetic field, on the one hand, the detection of the second linear magnetic field allows for a detection of the y-axis direction on the target coordinate axes, similarly to the detection of the first linear magnetic field, and on the other hand, a distance between the second linear magnetic field generator 10 and the capsule endoscope 2 is calculated based on the magnetic field strength.

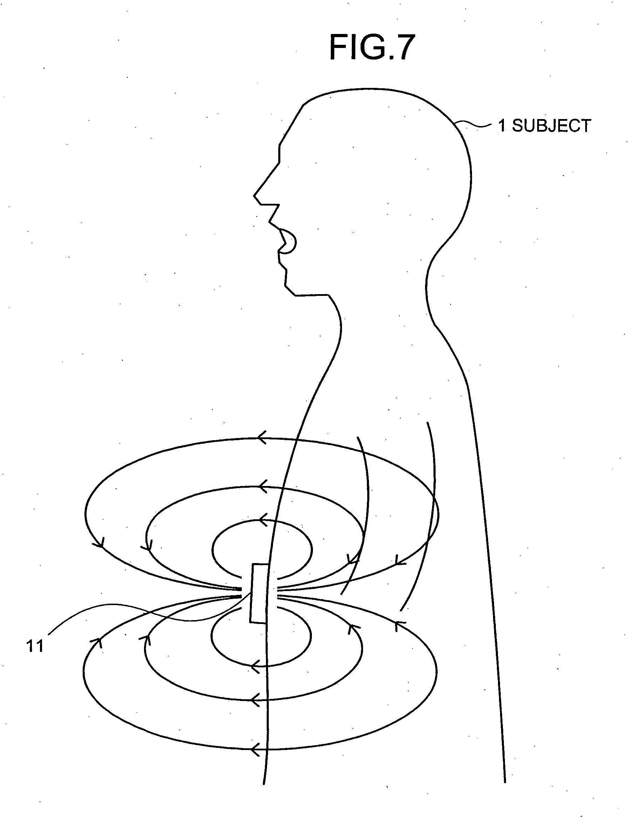

FIG. 7 is a schematic drawing of the diffuse magnetic field generated by the diffuse-magnetic-field generator 11. As shown also in FIG. 3, the coil 34 provided in the diffuse-magnetic-field generator 11 is formed in a spiral shape on a surface of the subject 1. As shown in FIG. 7, the magnetic field lines of the diffuse magnetic field generated by the coil 34 (not shown in FIG. 7) of the diffuse-magnetic-field generator 11 are once radially diffused, and then come back to the coil 34. The diffuse magnetic field is used to calculate the origin of the target coordinate axes on the reference coordinate axes, as described hereinafter.

An operation to detect the positional relationship of the target coordinate axes, which is fixed with respect to the capsule endoscope 2, with respect to the reference coordinate axes in the positional relationship detecting system according to the first embodiment is explained. A calculation process at the processor 12 is mainly explained hereinafter, and among the detection of the positional relationships, the calculation of the orientation of the target coordinate axes with respect to the reference coordinate axes and the calculation of the origin of the target coordinate axes on the reference coordinate axes are sequentially explained.

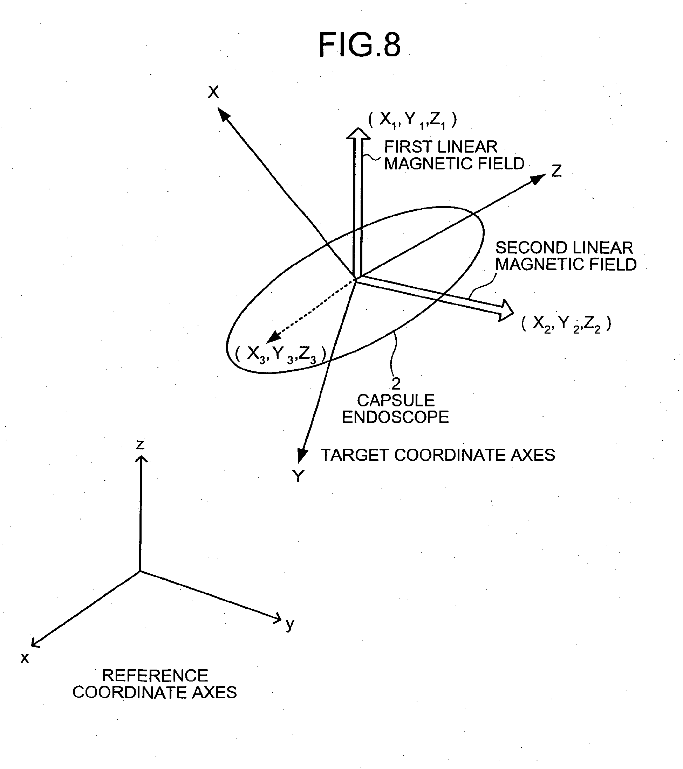

An orientation calculating operation performed by the orientation calculator 40 provided in the processor 12 is explained. FIG. 8 is a schematic drawing illustrating a relationship between the reference coordinate axes and the target coordinate axes during the traveling of the capsule endoscope 2 inside the subject 1. As explained hereinbefore, the capsule endoscope 2 travels along the passage inside the subject 1, and is rotated on the axis of the traveling direction by a predetermined angle. Therefore, as shown in FIG. 8, the target coordinate axes fixed with respect to the capsule endoscope 2 are misaligned with the reference coordinate axes fixed with respect to the subject 1.

The first linear magnetic field generator 9 and the second linear magnetic field generator 10 are each fixed with respect to the subject 1. Hence, the first and the second linear magnetic fields generated by the first linear magnetic field generator 9 and the second linear magnetic field generator 10, respectively, travel in certain directions with respect to the reference coordinate axes. Specifically, the first linear magnetic field travels in the z-axis direction and the second linear magnetic field travels in the y-axis direction of the reference coordinate axes. Therefore, the directions of the first and the second linear magnetic fields on the target coordinate axes correspond to the z-axis direction and the y-axis direction of the reference coordinate axes respectively, and in the first embodiment, the orientation of the target coordinate axes with respect to the reference coordinate axes is calculated based on the first and the second linear magnetic fields.

Specifically, the orientation is calculated as described hereinafter. The directions of the first and the second linear magnetic fields supplied in a time-sharing manner are detected by the magnetic field sensor 16 provided in the capsule endoscope 2. As described hereinbefore, the magnetic field sensor 16 is arranged so as to be fixed with respect to the capsule endoscope 2, and includes three axial sensors detecting magnetic field components in X-axis direction, Y-axis direction, and Z-axis direction, respectively. Thus, the magnetic field sensor 16 detects the directions of the first and the second linear magnetic fields on the target coordinate axes and transmits the detection result to the positional relationship detecting apparatus 3 through the radio transmitting unit 19.

The positional relationship detecting apparatus 3 receives the radio signals through the receiving antennas 7a to 7d, and performs a predetermined processing on the received radio signals in the receiving circuit 38 and the signal processing unit 39. Then, the signal processing unit 39 outputs the magnetic field signals S1 and S2 to the orientation calculator 40 as shown in FIG. 4. The magnetic field signals S1 reflects the detection result of the first linear magnetic field, and the magnetic field signal S2 reflects the detection result of the second linear magnetic field. In an example of FIG. 8, for example, the magnetic field signal S1 contains information on a coordinate (X₁, Y₁, Z₁) as the direction of the first linear magnetic field, and the magnetic field signal S2 contains information on a coordinate (X₂, Y₂, Z₂) as the direction of the second linear magnetic field.

On receiving the magnetic field signals S1 and S2, the orientation calculator 40 calculates the orientation of the target coordinate axes with respect to the reference coordinate axes. As described hereinbefore, the direction of the first linear magnetic field corresponding to the z-axis direction of the reference coordinate axes is represented by (X₁, Y₁, Z₁) on the target coordinate axes, and the direction of the second linear magnetic field corresponding to the y-axis direction is represented by (X₂, Y₂, Z₂) on the target coordinate axes. The orientation calculator 40 takes in the directions of the z-axis and the y-axis on the target coordinate axes, and also takes in the direction of the x-axis orthogonal to both of the z-axis and the y-axis based on the above mentioned correspondence. Specifically, when a coordinate whose inner products with (X₁, Y₁, Z₁) and with (X₂, Y₂, Z₂) are zero is represented as (X₃, Y₃, Z₃), the orientation calculator 40 takes in the coordinate (X₃, Y₃, Z₃) as a coordinate corresponding to the direction of the z-axis of the reference coordinate axes.

Thus, the orientation calculator 40 takes in the directions of the x-, y-, and z-axes on the target coordinate axes. The orientation calculation can be finished by taking in the aforementioned correspondences only; in the first embodiment, however, the orientation of the target coordinate axes is further calculated based on the reference coordinate axes. Specifically, the orientation calculator 40 in the first embodiment performs a predetermined coordinate transformation processing based on the aforementioned correspondence. The orientation calculator 40 calculates coordinates corresponding to the X-, Y-, and Z-axes of the target coordinate axes on the reference coordinate axes, and outputs the calculated coordinates as the orientation information. By performing the aforementioned coordinate transformation processing, it can be determined, for example, which direction on the reference coordinate axes corresponds to the Z-axis corresponding to the traveling direction of the capsule endoscope 2, and the traveling direction of the capsule endoscope 2 with respect to the subject 1 and the like can be recognized.

A calculation, which is performed by the position calculator 41, of the origin of the target coordinate axes on the reference coordinate axes is explained. Since the capsule endoscope 2 performs the imaging and the like of the subject interior image while traveling inside the subject 1, the origin of the target coordinate axes fixed with respect to the capsule endoscope 2 continually changes on the reference coordinate axes fixed with respect to the subject 1. Hence, the positional relationship detecting system according to the first embodiment calculates not only the orientation of the target coordinate axes but also the origin of the target coordinate axes. Hereinafter, the position calculation of the origin is explained in details.

Information used during the position calculating operation performed by the position calculator 41 is briefly explained. As shown in FIG. 4, the position calculator 41 receives the magnetic field signals S2 and S3 from the signal processing unit 39 and the orientation information from the orientation calculator 40. Further, the position calculator 41 retrieves the information stored in the magnetic-field line orientation database 42 as necessary. As described hereinbefore, the magnetic field signal S2 has information on the coordinate, which represents the direction of the second linear magnetic field detected by the magnetic field sensor 16, on the target coordinate axes, and in the example shown in FIG. 8, the magnetic field signal S2 contains information on the coordinate (X₂, Y₂, Z₂). The magnetic field signal S3 has information on the coordinate, which represents the direction of the diffuse magnetic field generated by the diffuse-magnetic-field generator 11 and detected by the magnetic field sensor 16, on the target coordinate axes. The orientation information input from the orientation calculator 40 specifically represents the directions of the X-, Y-, and Z-axes of the target coordinate axes on the reference coordinate axes, and information stored in the magnetic-field line orientation database 42 is a description on relationship between direction of the diffuse magnetic field and position in a region apart from the coil 32, which is provided in the second linear magnetic field generator 10, by a distance r.

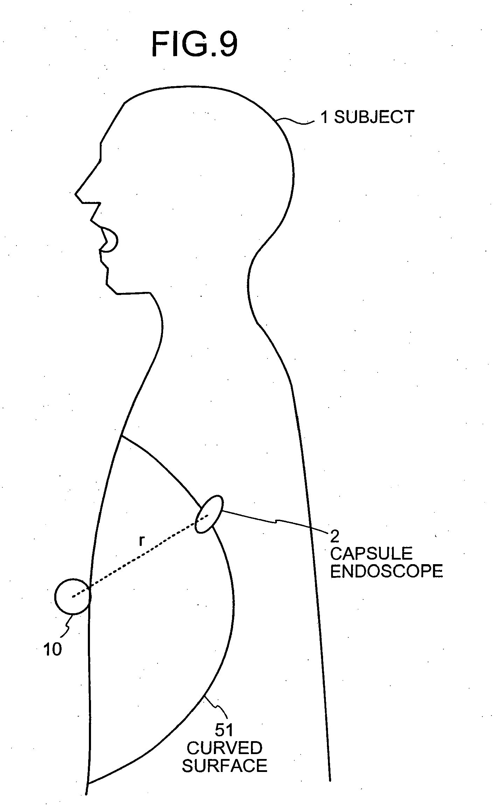

The position calculator 41 calculates the distance r between the capsule endoscope 2 and the second linear magnetic field generator 10 based on the magnetic field signal S2 among the aforementioned information. As described hereinbefore, the coil 32 provided in the second linear magnetic field generator 10 is arranged outside of the subject 1. Consequently, in the subject 1, the strength of the second magnetic field gradually decreases with distance from the coil 32 increases. Thus, the magnetic field strength and the distance from the coil 32 are correlated to each other. The position calculator 41 calculates a detected strength of the second linear magnetic field at a position of the capsule endoscope 2 based on the magnetic field signal S2, and calculates the distance r between the second linear magnetic field generator 10 (coil 32 to be precise) and the capsule endoscope 2 based on the calculated magnetic field strength. As a result, it becomes clear that the origin of the target coordinate axes fixed with respect to the capsule endoscope 2 is positioned on a curved surface 51 formed by assembly of points that are distance r away from the second linear magnetic field generator 10, as shown in FIG. 9.

Then, the position calculator 41 calculates the origin of the target coordinate axes on the curved surface 51. The position calculator 41 first calculates the direction of the diffuse magnetic field in the region where the capsule endoscope 2 is positioned based on the magnetic field signal S3 supplied from the signal processing unit 39 and the orientation information calculated by the orientation calculator 40. The magnetic field signal S3 reflects the result of detection of the diffuse magnetic field by the magnetic field sensor 16, and the magnetic field signal S3 contains information on the direction of the diffuse magnetic field on the target coordinate axes. Hence, the position calculator 41 calculates the direction of the diffuse magnetic field on the reference coordinate axes at the position of the capsule endoscope 2, by extracting the direction of the diffuse magnetic field on the target coordinate axes from the magnetic field signal S3, and by performing the coordinate transformation on the direction of the diffuse magnetic field based on the orientation information.

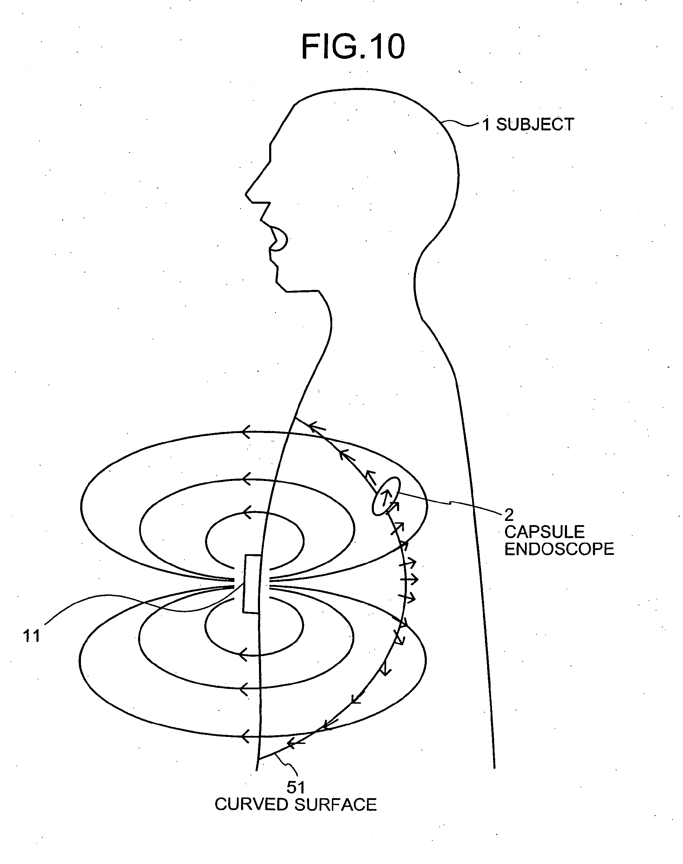

Then, the position calculator 41 calculates the origin of the target coordinate axes on the curved surface 51 by referring to the information stored in the magnetic-field line orientation database 42 based on the calculated direction of the diffuse magnetic field. FIG. 10 is a schematic drawing visually displaying the information stored in the magnetic-field line orientation database 42. As shown in FIG. 10, the diffuse magnetic field generated by the diffuse-magnetic-field generator 11 is different from the linear magnetic field in that the direction thereof has positional dependence. Hence, the direction of the diffuse magnetic field on the curved surface 51 varies from position to position. The magnetic-field line orientation database 42 stores the correspondence between the direction of the diffuse magnetic field and the position on the curved surface 51. Hence, the position calculator 41 refers to the magnetic-field line orientation database 42 based on the direction of the diffuse magnetic field as derived based on the magnetic field signal S3 and the like, to calculate the position, which corresponds to the origin of the target coordinate axes fixed with respect to the capsule endoscope 2, on the reference coordinate axes, and to output the positional information related with the calculated position. By performing the aforementioned operation, the detection of the positional relationship of the target coordinate axes with respect to the reference coordinate axes is finished, and the orientation information calculated by the orientation calculator 40 and the position information calculated by the position calculator 41 are output to the memory 43. Then, the memory 43 stores the image signals S4 corresponding to the subject interior image and the orientation information and the position information associated with the image signals S4 in the portable recording medium 5.

As described hereinbefore, in the positional relationship detecting system according to the first embodiment, the receiving antenna 7 and the transmitting antenna 8 are selected by the selection controller 48 based on the detected positional relationship. Hereinafter, a control operation of the selection controller 48 is explained by using an example in which the receiving antenna 7 is selected by using the receiving antenna selector 37. The selection controller 48 stores a position and an orientation direction of the transmitting antenna 27, which is provided in the capsule endoscope 2, on the target coordinate axes in advance, and receives the orientation information and the position information from the orientation calculator 40 and the position calculator 41, respectively. Then, the selection controller 48 converts the position and the orientation direction of the transmitting antenna 27 on the target coordinate axes to values on the reference coordinate axes based on the orientation information and the position information, and takes in the position and the orientation direction of the transmitting antenna 27 on the reference coordinate axes. Then, the selection controller 48 extracts the receiving antenna 7, from the receiving antennas 7a to 7h, most appropriate for receiving the radio signals transmitted from the transmitting antenna 27 based on the taken-in position and the orientation direction of the transmitting antenna 27. Then, the selection controller 48 sends a command to the receiving antenna selector 37 to select the extracted receiving antenna 7. Based on the command, the receiving antenna selector 37 selects the receiving antenna 7, and the reception of the radio signals through the selected receiving antenna 7 is started.

The aforementioned selection mechanism is applied similarly to the selection of the transmitting antenna 8. When the transmitting antenna 8 is to be selected, the selection controller 48 calculates the position and the like of the receiving antenna 28 on the reference coordinate axes based on previously stored position and the orientation direction of the receiving antenna 28, which is provided in the capsule endoscope 2, on the target coordinate axes, the supplied orientation information, and the supplied position information. Then, the selection controller 48 extracts the transmitting antenna 8 most appropriate for the radio transmission with respect to the receiving antenna 28 based on the calculated result, and sends a command corresponding to the extraction result to the transmitting antenna selector 47, thereby performing the selection of the transmitting antenna 8.

Advantages associated with the positional relationship detecting system according to the first embodiment are explained. The positional relationship detecting system according to the first embodiment detects the magnetic field generated by the first linear magnetic field generator 9 and the like by utilizing the magnetic field sensor 16 provided in the capsule endoscope 2, and calculates the positional relationship of the target coordinate axes with respect to the reference coordinate axes based on the result of the aforementioned detection of the positional relationship. Theoretically, the positional relationship can be calculated based on the magnetic field, which is generated by the mechanism inside the capsule endoscope 2 and detected by the magnetic field sensor provided outside. However, by employing the configuration in which the magnetic field generated by the elements provided outside of the subject 1 is detected by the magnetic field sensor 16, the positional relationship detecting system according to the present first embodiment obtains an advantage in which the configuration of the capsule endoscope 2 can be simplified.

When the magnetic field is generated by the elements provided in the capsule endoscope 2, it is necessary to provide a magnetic field shielding mechanism and the like to avoid an effect of the strong magnetic field, which is generated by the magnetic field generating mechanism, on the operation of the radio transmitting unit 19 and the like. In the first embodiment, the magnetic field is generated by the elements such as the first linear magnetic field generator 9 and the like provided outside of the capsule endoscope 2. Consequently, negative influence, which is caused by the magnetic field, on the operation of the elements such as the radio transmitting unit 19 provided in the capsule endoscope 2 can practically be ignored, and it is unnecessary to separately provide elements such as the magnetic field shielding mechanism. Therefore, by employing the configuration in which the external mechanism generates the magnetic field, the configuration of the capsule endoscope 2 can be simplified.

The positional relationship detecting system according to the first embodiment calculates the positional relationship based on the directions of the plurality of the linear magnetic fields on each of the reference coordinate axes and the target coordinate axes. When, for example, the orientation of the target coordinate axes is calculated by using a single linear magnetic field, it is difficult to clearly define the single orientation of the target coordinate axes. However, it is apparent from the aforementioned explanation that the orientation of the target coordinate axes can accurately be detected by employing the configuration in which the orientation is detected based on the plurality of the linear magnetic fields.

In the first embodiment, the positional dependence of the direction of the diffuse magnetic field is utilized to calculate the origin of the target coordinate axes. The origin of the target coordinate axes with respect to the reference coordinate axes can be calculated by, for example, providing plural magnetic field generating sources, for example, three magnetic field generating sources, that have a function of generating a magnetic field whose strength decreases with distance and that are fixed on the reference coordinate axes. However, the number of the magnetic field generating sources can be reduced by utilizing the diffuse-magnetic-field generator 11, as described in the present first embodiment. At least theoretically, the position can be calculated with some level of accuracy by providing the single diffuse-magnetic-field generator 11 which generates the diffuse magnetic field whose direction has positional dependence, and the positional relationship detecting system according to the first embodiment obtains an advantage in which the number of the magnetic field generating mechanisms necessary to detect the position can be reduced.

The first embodiment employs a configuration in which the property, i.e., the distance dependent attenuation property, of the magnetic field generated by the second linear magnetic field generator 10 can be utilized for the detection of the origin of the target coordinate axes, whereby more accurate position detection is allowed. There is a problem in which it is difficult to realize a mechanism generating the diffuse magnetic field whose direction has an absolute positional dependence in practice. For example, the diffuse-magnetic-field generator 11 according to the first embodiment is parallel to the yz plane on the reference coordinate axes, and the direction of the diffuse magnetic field on the plane region including the coil 34 is parallel to the x-axis anywhere. In such a configuration, it is difficult to accurately detect the position based only on the direction of the diffuse magnetic field. Therefore, in the first embodiment, the distance between the second linear magnetic field generator 10 and the capsule endoscope 2 is also used to detect the position, so that the position of the origin can be detected more accurately.

In the first embodiment, the second linear magnetic field generator 10 and the diffuse-magnetic-field generator 11 can be arranged at positions close to each other. For example, when the origin of the target coordinate axes is detected by utilizing three magnetic field generating mechanism generating the magnetic field whose strength decreases with distance as described hereinbefore, it is preferable to separate each of the magnetic field generating mechanism by a predetermined distance, to improve the accuracy of the position detection. On the other hand, in the first embodiment, since the second linear magnetic field and the diffuse magnetic field are used for the position detection based on different perspectives, a correlation between a distance, which is between the position of,the second linear magnetic field generator 10 and the position of the diffuse-magnetic-field generator 11, and the accuracy of the position detection of the origin of the target coordinate axes is extremely low. Therefore, in the first embodiment, the second linear magnetic field generator 10 and the diffuse-magnetic-field generator 11, for example, can be formed on, for example, the same substrate, so that an advantage in which the configuration of the system is simplified is obtained.

### Second Embodiment

A positional relationship detecting system according to a second embodiment is explained. The positional relationship detecting system according to the second embodiment uses Earth magnetism as the first linear magnetic field, and the first linear magnetic field generator is not provided corresponding to the use of the Earth magnetism as the first linear magnetic field.

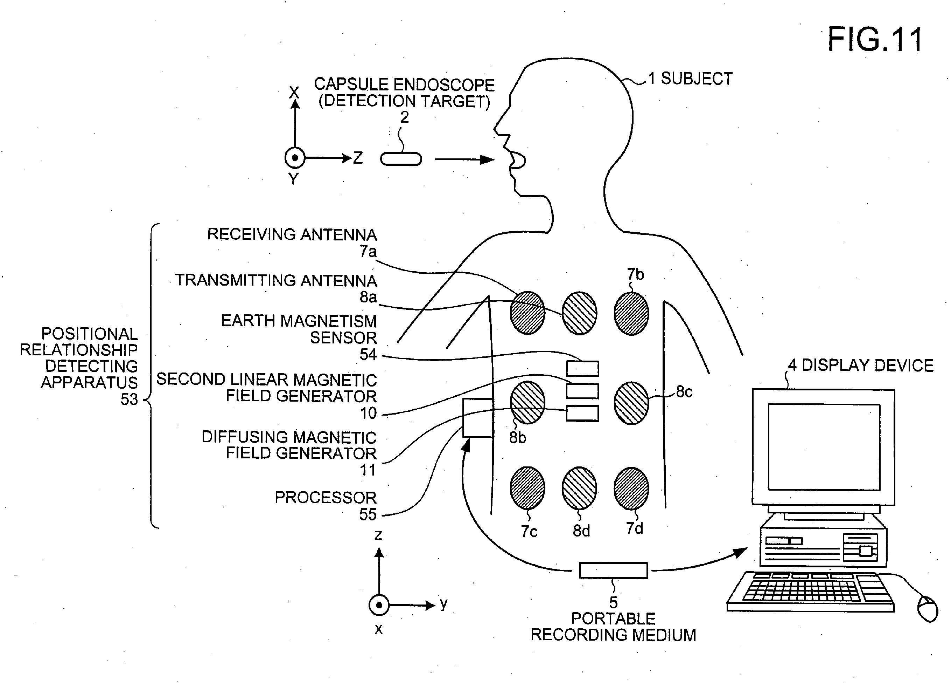

FIG. 11 is a schematic drawing of an overall configuration of the positional relationship detecting system according to the present second embodiment. In the explanation below, elements with names and numbers that are the same as those of the first embodiment have the same configurations and functions as those of the first embodiment unless otherwise specified hereinbelow.

As shown in FIG. 11, in the positional relationship detecting system according to the second embodiment, a positional relationship detecting apparatus 53 additionally includes an Earth magnetism sensor 54 for detecting a direction of the Earth magnetism, and in a processor 55, the direction of the Earth magnetism on the reference coordinate axes is calculated based on the detection result of the Earth magnetism sensor 54.

The Earth magnetism sensor 54 basically has a same configuration as the configuration of the magnetic field sensor 16 provided in the capsule endoscope 2. In a region where the Earth magnetism sensor 54 is arranged, the Earth magnetism sensor 54 detects strength of magnetic field components that are each associated with different directions of predetermined three axes, and outputs electronic signals corresponding to the detected magnetic field strength. Further, unlike the magnetic field sensor 16, the Earth magnetism sensor 54 is arranged on a body surface of the subject 1, and detects the strength of the magnetic field components corresponding to the x-, y-, and z-axes of the reference coordinate axes fixed with respect to the subject 1. The Earth magnetism sensor 54 detects the direction of the Earth magnetism, and outputs the electronic signals corresponding to the magnetic field strength detected corresponding to the x-, y-, and z-axes directions.

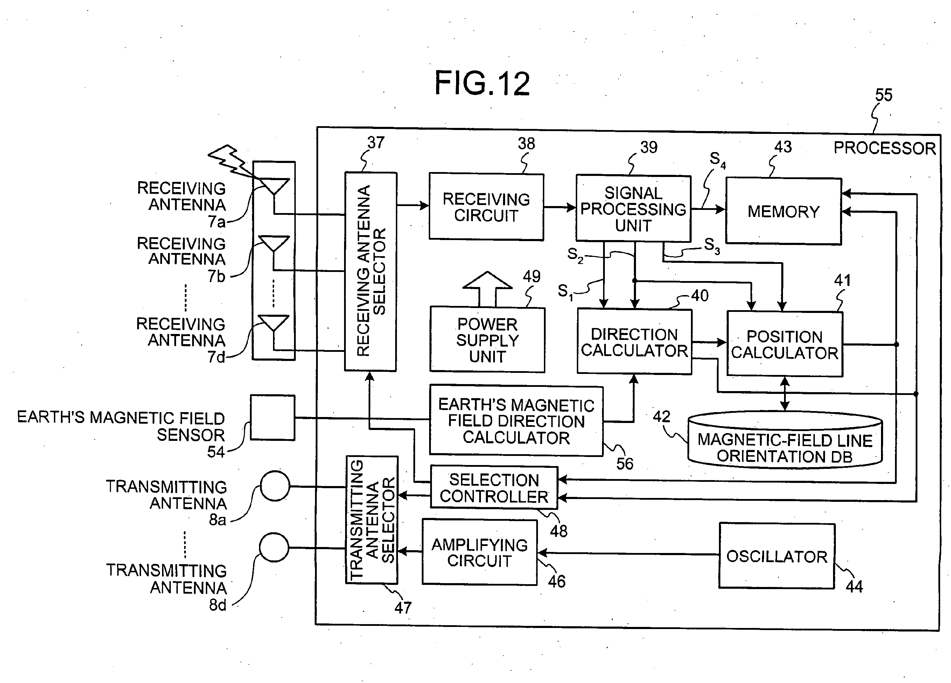

The processor 55 according to the second embodiment is explained. FIG. 12 is a block diagram of a configuration of the processor 55. As shown in FIG. 12, the processor 55 basically has the same configuration as the configuration of the processor 12 according to the first embodiment. On the other hand, the processor 55 has an Earth magnetism orientation calculator 56 that calculates the direction of the Earth magnetism on the reference coordinate axes based on the electronic signals supplied from the Earth magnetism sensor 54, and outputs the calculated result to the orientation calculator 40.

It is necessary to consider the calculation of the direction of the Earth magnetism on the reference coordinate axes fixed with respect to the subject 1 when the Earth magnetism is used as the first linear magnetic field. Since the subject 1 can freely move while the capsule endoscope 2 travels inside the body, the positional relationship between the Earth magnetism and the reference coordinate axes fixed with respect to the subject 1 changes according to the motion of the subject 1. In view of the calculation of the positional relationship of the target coordinate axes with respect to the reference coordinate axes, there is a problem in which a correspondence between the reference coordinate axes and the target coordinate axes with respect to the direction of the first linear magnetic field cannot be revealed when the direction of the first linear magnetic field on the reference coordinate axes becomes unknown.

Therefore, in the present second embodiment, the Earth magnetism sensor 54 and an Earth magnetism orientation calculator 56 are provided to monitor the direction of the Earth magnetism, which changes on the reference coordinate axes due to the motion and the like of the subject 1. The Earth magnetism orientation calculator 56 calculates the direction of the Earth magnetism on the reference coordinate axes based on the detection result of the Earth magnetism sensor 54, and outputs the calculated result to the orientation calculator 40. The orientation calculator 40 calculates a correspondence between the reference coordinate axes and the target coordinate axes with respect to the direction of the Earth magnetism by using the direction of the Earth magnetism, and calculates the orientation information together with the correspondence according to the second linear magnetic field.

The direction of the Earth magnetism and the second linear magnetic field generated by the second linear magnetic field generator 10 sometimes become parallel to each other corresponding to the direction of the subject 1. Even then, the positional relationship can be detected by using data corresponding to the orientation and the origin of the target coordinate axes right before the Earth magnetism and the second magnetic field become parallel to each other. Further, to avoid the Earth magnetism and the second linear magnetic field being parallel to each other, the coil 34 configuring the second linear magnetic field generator 10 can be extended in, for example, the z-axis direction of the reference coordinate axes instead of extending the coil 34 in the y-axis direction as shown in FIG. 3.

An advantage associated with the positional relationship detecting system according to the second embodiment is explained. In addition to the advantages associated with the first embodiment, the positional relationship detecting system according to the second embodiment obtains an advantage by using the Earth magnetism. The mechanism generating the first linear magnetic field can be removed by employing the configuration using the Earth magnetism as the first linear magnetic field, and the positional relationship of the target coordinate axes with respect to the reference coordinate axes can be calculated while reducing load on the subject 1 at the time of insertion of the capsule endoscope 2 into the body. Since the Earth magnetism sensor 54 can be realized with a MI sensor and the like, the capsule endoscope can be sufficiently miniaturized. Hence, the load on the subject 1 does not increase due to additionally providing the Earth magnetism sensor 54.

The use of the Earth magnetism as the first linear magnetic field is advantageous in terms of reduced power consumption. When the first linear magnetic field is generated by using the coil and the like, power consumption is increased due to the current and the like running through the coil. However, by using the Earth magnetism, power is not required for such an element. Hence, a system with low power consumption can be realized.

As described hereinbefore, the present invention is explained corresponding to the first and the second embodiments; however, the present invention is not limited to the first and the second embodiments. Hence, various embodiments and modifications are readily occurred to those skilled in the art. For example, a plurality of magnetic field generators generating the linear magnetic field or the diffuse magnetic field whose strength decreases with distance may be used as a mechanism to calculate the origin of the target coordinate axes, instead of the magnetic field generators of the embodiments. The origin of the target coordinate axes can be detected by previously taking in the position of the plurality of the magnetic field generators on the reference coordinate system, and by calculating the distance between the plurality of the magnetic field generators and the capsule endoscope 2 based on the magnetic field strength detected by the capsule endoscope 2.

In the first and the second embodiments, the reference coordinate axes and the target coordinate axes are defined based on an orthogonal three dimensional coordinate system. It should be obvious, however, that the reference coordinate axes and the like are not necessarily be limited to the orthogonal three dimensional coordinate system. Hence, the reference coordinate axes and the like can be defined by, for example, a three dimensional polar coordinate system. Alternatively, the reference coordinate axes may be defined by a two dimensional coordinate system or one dimensional coordinate system depending on the use of the system.

### INDUSTRIAL APPLICABILITY

As described hereinbefore, a positional relationship detecting apparatus and a positional relationship detecting system according to the present invention are useful to calculate a positional relationship between target coordinate axes fixed with respect to a detection target and reference coordinate axes defined independently of motion and the like of the detection target, and particularly suitable for capsule endoscope.

## Claims

1. A positional relationship detecting apparatus that detects a positional relationship between a target coordinate axis fixed with respect to a detection target and a reference coordinate axis defined independently of motion of the detection target, the positional relationship detecting apparatus comprising:
an orientation calculator that calculates an orientation of the target coordinate axis with respect to the reference coordinate axis based on correspondences, one correspondence between a direction of a first linear magnetic field having a predetermined direction on the target coordinate axis and a direction of the first linear magnetic field on the reference coordinate axis, other correspondence between a direction of a second linear magnetic field different from the first linear magnetic field on the target coordinate axis and a direction of the second linear magnetic field on the reference coordinate axis.

2. The positional relationship detecting apparatus according to claim 1, further comprising:
a first linear magnetic field generator that is arranged at a predetermined position on the reference coordinate axis and generates the first linear magnetic field; and
a second linear magnetic field generator that is arranged at a predetermined position on the reference coordinate axis and generates the second linear magnetic field, wherein
the orientation calculator calculates the orientation of the target coordinate axis with respect to the reference coordinate axis based on the directions, which are detected by the detection target, of the first and the second linear magnetic fields on the target coordinate axis and the directions of the first and the second linear magnetic fields on the predetermined reference coordinate axis.

3. The positional relationship detecting apparatus according to claim 1, further comprising:
a second linear magnetic field generator that is arranged at a predetermined position on the reference coordinate axis and generates the second linear magnetic field; and
a magnetic field sensor that detects the direction of the first linear magnetic field on the reference coordinate axis, wherein
the first linear magnetic field is formed by Earth magnetism, and
the orientation calculator calculates the orientation of the target coordinate axis with respect to the reference coordinate axis based on the direction, which is detected by the magnetic field sensor, of the first linear magnetic field on the reference coordinate axis, the direction of the second linear magnetic field on the predetermined reference coordinate axis, and directions, which are detected by the detection target, of the first and the second linear magnetic fields on the target coordinate axis.

4. The positional relationship detecting apparatus according to claim 1, further comprising:
a position calculator that calculates an origin of the target coordinate axis with respect to the reference coordinate axis based on a direction of a diffuse magnetic field whose direction has positional dependence at a position of the detection target, and the orientation, which is calculated by the orientation calculator, of the target coordinate axis with respect to the reference coordinate axis.

5. The positional relationship detecting apparatus according to claim 2, further comprising:
a position calculator that calculates an origin of the target coordinate axis with respect to the reference coordinate axis based on a direction of a diffuse magnetic field whose direction has positional dependence at a position of the detection target, and the orientation, which is calculated by the orientation calculator, of the target coordinate axis with respect to the reference coordinate axis.

6. The positional relationship detecting apparatus according to claim 3, further comprising:
a position calculator that calculates an origin of the target coordinate axis with respect to the reference coordinate axis based on a direction of a diffuse magnetic field whose direction has positional dependence at a position of the detection target, and the orientation, which is calculated by the orientation calculator, of the target coordinate axis with respect to the reference coordinate axis.

7. The positional relationship detecting apparatus according to claim 5, wherein
the second linear magnetic field has a property in which a magnetic field strength decreases corresponding to a distance from the second linear magnetic field generator, and
the position calculator calculates the origin of the target coordinate axis with respect to the reference coordinate axis by calculating and using a distance between the detection target and the second linear magnetic field generator based on the magnetic field strength of the second linear magnetic field at the position of the detection target.

8. The positional relationship detecting apparatus according to claim 6, wherein
the second linear magnetic field has a property in which a magnetic field strength decreases corresponding to a distance from the second linear magnetic field generator, and
the position calculator calculates the origin of the target coordinate axis with respect to the reference coordinate axis by calculating and using a distance between the detection target and the second linear magnetic field generator based on the magnetic field strength of the second linear magnetic field at the position of the detection target.

9. A positional relationship detecting system, comprising:
a detection target defined with a predetermined target coordinate axis; and
a positional relationship detecting apparatus that detects a positional relationship between the target coordinate axis and a reference coordinate axis defined independently of motion of the detection target, wherein
the detection target includes
a magnetic field sensor that detects a magnetic field generated in a region where the detection target is positioned, and
a radio signal transmitter that transmits a radio signal containing information on the magnetic field detected by the magnetic field sensor, and
the positional relationship detecting apparatus includes
a magnetic field generator that generates a magnetic field in the region where the detection target is positioned, and
an orientation calculator that calculates an orientation of the target coordinate axis with respect to the reference coordinate axis based on the radio signal transmitted from the detection target.

10. The positional relationship detecting system according to claim 9, wherein
the magnetic field generator includes
a first linear magnetic field generator that is arranged at a predetermined position on the reference coordinate axis and generates a first magnetic field in a predetermined direction, and
a second linear magnetic field generator that is arranged at a predetermined position on the reference coordinate axis and generates a second linear magnetic field different from the first linear magnetic field, and
the orientation calculator calculates the orientation of the target coordinate axis with respect to the reference coordinate axis based on correspondences, one correspondence between a direction of the first linear magnetic field on the target coordinate axis and a direction of the first linear magnetic field on the reference coordinate axis, other correspondence between a direction of the second linear magnetic field on the target coordinate axis and a direction of the second linear magnetic field on the reference coordinate axis.

11. The positional relationship detecting system according to claim 9, wherein
the magnetic field generator further includes a diffuse-magnetic-field generator that generates a diffuse magnetic field whose direction has positional dependence, and
the positional relationship detecting apparatus includes a position calculator that calculates an origin of the target coordinate axis on the reference coordinate axis by using the positional dependence of the direction of the diffuse magnetic field.

12. The positional relationship detecting system according to claim 10, wherein
the magnetic field generator further includes a diffuse-magnetic-field generator that generates a diffuse magnetic field whose direction has positional dependence, and
the positional relationship detecting apparatus includes a position calculator that calculates an origin of the target coordinate axis on the reference coordinate axis by using the positional dependence of the direction of the diffuse magnetic field.
